# EUROPEAN PATENT SPECIFICATION

(11) **EP 4 368 137 B1**
(45) Date of publication and mention of the grant of the patent: **14.10.2026**
(21) Application number: 22206105.3
(22) Date of filing: 08.11.2022
(51) Int. Cl.: A61B 34/20

(54) **TECHNIQUE FOR DETERMINING A SURFACE REGISTRATION BASED ON MECHANICALLY ACQUIRED TISSUE SURFACE DATA**
VERFAHREN ZUR BESTIMMUNG EINER OBERFLÄCHENREGISTRIERUNG AUF DER BASIS VON MECHANISCH ERFASSTEN GEWEBEOBERFLÄCHENDATEN
TECHNIQUE DE DÉTERMINATION D'UN ENREGISTREMENT DE SURFACE SUR LA BASE DE DONNÉES DE SURFACE DE TISSU ACQUISES MÉCANIQUEMENT

(43) Date of publication of application: 15.05.2024
(73) Proprietor: Stryker European Operations Limited, Carrigtwohill, Co. Cork T45 HX08 (IE)
(72) Inventor: Dr. KÄSEBERG, Marc, 16359 Biesenthal (DE); WINNE, Christian, 13127 Berlin (DE)
(74) Representative: Röthinger, Rainer

(56) References cited:
- US-A1- 2006 036 397
- US-A1- 2019 015 163
- US-B2- 10 660 707

## Description

### Technical Field

The present disclosure generally relates to the field of computer-assisted surgery. In particular, a technique for determining a surface registration is presented. The technique presented herein can be practiced in the form of a method, a computer program product, an apparatus and a system.

### Background

Many computer-assisted surgical procedures are performed based on a registration between preoperatively acquired data associated with a patient anatomy and intraoperatively acquired data associated with the patient anatomy. Such registrations may comprise image-to-image or image-to-physical transformations. One commonly used image-to-physical transformation is a surface registration which requires acquiring a multitude of points on a tissue surface of the patient anatomy and a corresponding surface model derived from image data.

The acquisition of the points on the patient anatomy surface may be performed in different ways. For example, contact-free techniques like scanning the surface with a laser range scanner may be utilized. Other approaches include contact-involving techniques like attaching trackable markers to the surface or touching the surface with a tracked pointing device.

US 2006/0036397 A1 discloses a method for identifying the location of at least one characteristic point of a body structure. The method includes identifying the location of a plurality of points in an area of the at least one characteristic point, and ascertaining the location of the at least one characteristic point from the location of the plurality of points. the step of ascertaining the location of the at least one characteristic point from the location of the plurality of points may include a step of ascertaining the at least one characteristic point to be below the skin surface a distance substantially equal to a thickness of the skin layer and any intermediate layers. Thus, by compensating for the thickness of the skin, the position of one or more characteristic points can be used to register a bone structure.

In many surgical cases, for example tumour treatment, the patient anatomy comprises soft tissue (e.g., a breast, a liver or skin). In contact-involving techniques, especially when the surface is touched with a pointing device, such soft tissues are deformed. Due to the touch-induced deformation, an error is introduced in the registration since the corresponding surface model derived, for example, from image data is not deformed. Further, the touch-induced deformation is generally not homogenous over the soft tissue surface, since a softness of the tissue and the force applied by the pointing device may vary over the surface. Thus, the registration error also depends on the performance of a user when operating the pointing device. Since surgical instruments used in a computer-assisted surgical procedure are often navigated based on the registration, each registration error increases the health risk for the patient. Therefore, it is a general goal to reduce registration errors as much as possible.

To reduce the registration error introduced due to touch-induced deformation, a user is commonly trained to touch points on the soft tissue surface that are known to be less deformable than other points, e.g., due to directly underlying hard tissue like bone. However, relying on the training of users requires a high cognitive load from the users and is always prone to human error. Further, identifying less deformable points may be difficult for less experienced users.

US 10,660,707 B2 discloses a method including receiving a computerized tomography image comprising voxels of a body part of a subject, segmenting the image so as to identify a surface of a skin and a surface of a bone in the image, measuring respective minimum distances to the bone from a plurality of points on the surface of the skin, and rendering an image of the surface of the skin while visually coding the rendered image so as to indicate the respective minimum distances. A user may then touch with a registration probe the skin of the subject at bony areas as guided by the image.

US 11,276,169 B2 and US 6,560,354 B1 disclose registration techniques based on surface data acquired by touching a soft tissue surface at predefined locations. The predefined locations may be identified via markers attached to the surface or via anatomical landmarks automatically identified and displayed to a user. To reduce the registration error introduced due to touch-induced deformation, the acquired surface data are weighted for each touched location based on, e.g., a distance between the respective location and a bone closest thereto. In particular, soft tissue locations that are known to be less prone to deformation (e.g., having a smaller distance between the respective location and a bone closest thereto compared to other locations) may be associated with a higher weight for determining a registration as such locations are generally less deformable. However, defining predefined locations restricts a user in his or her selection of points to touch. Moreover, attaching markers requires additional hardware and generally increases the duration of a surgical procedure. US 2019/015163 A1 discloses registration of a bone model with the patient by touching points on the skin surface with a stylus.

It would thus be desirable to provide a technique that reduces a registration error introduced by a touch-induced deformation without overly relying on the user's experience or being restricted to predefined locations or the use of markers.

### Summary

There is a need for a technique for determining a surface registration between a first soft tissue surface defined based on mechanically acquired first surface data and a second soft tissue surface defined based on image data that addresses one or more of the above-mentioned problems or other problems. The invention is defined by appended claims 1, 14, and 15.

According to a first aspect, a computer-implemented method for determining a surface registration between a first soft tissue surface defined based on mechanically acquired first surface data and a second soft tissue surface defined based on image data is provided. The method comprises obtaining the first surface data, wherein the first surface data comprise a first set of points mechanically acquired by contacting the soft tissue with a pointing device and applying a correction model on the first surface data to obtain corrected first surface data, wherein the correction model is configured to shift relative positions of two or more points in the first set. The method also comprises determining a first surface registration between the first and the second soft tissue surfaces based at least in part on the corrected first surface data.

The pointing device may be a tracked probe (e.g., with a pen-like configuration). The tracked probe may be tracked optically or electromagnetically. A coordinate system of the tracked probe may be registered with a coordinate system of the (undeformed) soft tissue based on known registration techniques. For example, the tracked probe may be optically tracked and the probe may be registered to the coordinate system of the soft tissue based on image data indicative of the tracked probe and the soft tissue.

In some variants, the correction model may be configured to determine a correction vector for at least one of the points in the first set. The correction vector may define a shift relative to one or more further points in the first set. A respective correction vector may be determined for a single point in the first set, multiple points in the first set, or all points in the first set.

In some variants, the correction vector may be determined based at least in part on the registered coordinate systems of the tracked probe and the soft tissue. In some variants, an earlier surface registration may have been determined prior to the first surface registration and based at least in part on the first surface data. In such a case, the correction vector may be determined based at least in part on the earlier surface registration. The earlier surface registration may in some variants not take a soft-tissue deformation into account (and may thus be a fast, but coarse registration in comparison to the first registration).

In some variants, the correction vector may be oriented normal to the first soft tissue surface. The orientation of the correction vector may be determined based at least in part on the registered coordinate systems of the tracked probe and the soft tissue. Additionally, or in the alternative, the orientation of the correction vector may be determined based at least in part on the earlier surface registration.

In some variants, obtaining the first surface data may comprise determining, for at least one of the points in the first set, an angle between the pointing device and a surface normal of the first soft tissue surface when the pointing device contacts the soft tissue at the respective point. To this end, the pointing device may be tracked. The surface normal may be determined directly at the respective point or at one or more points on the first soft tissue surface located in an area surrounding the respective point. The surface normal may be determined based at least in part on image data indicative of the soft tissue, or on a soft tissue model or a combination thereof. The correction vector may be determined based at least in part on the angle determined for the point associated with the respective correction vector.

A maximum length of the correction vector may be predefined. The maximum length may be between 0.1 mm and 10 mm (e.g. between 0.5 mm and 5 mm).

At least one of the points in the first set may be associated with a respective point on a hard tissue surface located beneath the soft tissue surface. In these variants, obtaining the first surface data may comprise determining, for the at least one of the points in the first set, a distance between the respective point in the first set and the associated point on the hard tissue surface. The correction vector may be determined based at least in part on the distance determined for the point in the first set associated with the correction vector. The respective point on the hard tissue surface may be the point on the hard tissue surface that is closest to the associated point in the first set. The closest point may be determined automatically via an optimization algorithm.

In some variants, determining the correction vector may be based at least in part on a linear soft tissue deformation model that receives the distance as an input parameter. The linear soft tissue deformation model may define at least one of a minimum distance threshold below which no deformation takes place and a maximum distance threshold indicative of a maximum distance-based deformation.

In some variants, determining the correction vector may be based at least in part on a polynomial deformation model or a logarithmic deformation model. In some variants, additional information, e.g., a tissue type may be determined. The additional information may be determined based on, e.g., medical image data, e.g., CT scans of the soft tissue, and the correction vector may be determined based at least in part on the additional information. In some variants, a model may be determined based on the thickness of the soft tissue at respective points and the correction vector may be determined based at least in part on the determined model.

In some variants, a soft tissue model and a hard tissue model may be provided. The distance between the respective point in the first set and the associated point on the hard tissue surface may be obtained based at least in part on the soft tissue model and the hard tissue model. The soft tissue may comprise skin of a patient, e.g., facial skin, and the hard tissue may comprise a facial bone. In some variants, the distance between the respective point in the first set and the associated point on the hard tissue surface may be determined based at least in part on medical imaging data, e.g., based on a computer tomography (CT) or magneto resonance imaging (MRI) scan.

In some variants, multiple different correction models configured to shift relative positions of two or more points in the first set may be provided. The multiple different models may comprise models comprising the same parameters but with different parameter values, e.g., a different maximum length of the correction vector. The multiple different models may comprise models with different parameters. The method may further comprise applying each of the different correction models to the first surface data to obtain different versions of the corrected first surface data. The first surface registration may then be determined based at least in part on the different versions of the first surface data. In some variants, for example, a surface registration may be determined for each of the different versions of the first surface data, and the first surface registration may be determined based on respective registration accuracies of the surface registrations determined for the different versions of the first surface data. For example, the first registration may correspond to the registration with the highest registration accuracy out of the determined surface registrations. In some variants, the different correction models may be applied in parallel. Additionally, the respective registration accuracies may be determined in parallel. Determining a respective registration accuracy may comprise determining a distance between each registered point and the second soft tissue surface for the respective registration. Further, a registration error for each point of the respective registration may be determined based on the determined distance and the registration accuracy of the respective registration may be based at least in part on the mean error of the registered points.

The method may further comprise obtaining second surface data. The second surface data may comprise a second set of points mechanically acquired by contacting the soft tissue with a pointing device and applying the correction model on the second surface data to obtain corrected second surface data, wherein the correction model may be configured to shift relative positions of two or more points in the second set, and determining a second surface registration between the first and the second soft tissue surfaces based at least in part on the corrected first surface data, the corrected second surface data, and the first surface registration.

Additionally, the method may comprise reapplying the correction model on the first surface data to obtain revised corrected first surface data. The second surface registration may then be determined based at least in part on the revised corrected first surface data, the corrected second surface data, and the first surface registration.

In some variants, at least one further iteration of obtaining additional surface data, applying the correction model at least on the additional surface data, and determining an additional surface registration may be performed. For example, the method may comprise at least a third, fourth and fifth iteration. For each iteration, e.g., the second, third, fourth, fifth iteration, the additional surface data may be acquired in parallel to determining the surface registration of the preceding iteration, e.g., first, second, third, fourth iteration. For each iteration, the first soft tissue surface may be defined based on the additionally acquired data and the previously acquired data. For the example of the second iteration, the first soft tissue surface may be defined based on a combination of the second and the first surface data, and so on.

The method may comprise determining a registration accuracy of at least one of the registrations described herein, e.g., one or more of the earlier surface registration, the first surface registration, the second surface registration and any subsequent surface registration. The registration accuracy may be determined for multiple or all of the registrations described herein. In some variants, it may be determined to perform another iteration or to stop the method based on the registration accuracy. For example, in case the last determined registration accuracy fulfills a predefined threshold condition, no further iterations are needed and the method may be stopped. In another example, the determined registration accuracies of, e.g., two subsequent surface registrations may be compared to each other to determine a change in the registration accuracy of two subsequent surface registrations. Based on the change of the registration accuracy between the two subsequent registrations, e.g., based on a threshold of a difference between the registration accuracies, another iteration may be performed as described above. For example, the third iteration may be performed based on thresholding a difference between the registration accuracies of the first and the second registration.

In some variants registered surface data may be displayed to a user, e.g., a surgeon. The displayed registered data may be based on one of the determined surface registrations, e.g., the latest determined surface registration, the surface registration with the highest registration accuracy or any of the determined surface registrations having a registration accuracy that meets a predefined threshold condition. In some variants the displayed surface data may be updated in real time based at least in part on additionally acquired surface data and a subsequently determined registration. The resulting display may be used for surgical navigation purposes (e.g., to output navigation instructions for a surgical instrument to a user).

In some variants, the mechanically acquired points may define a first point cloud, and a second point cloud may be defined based on the image data defining the second soft tissue surface. Determining any of the surface registrations described herein may then be based at least in part on utilizing a point cloud registration algorithm, e.g., an iterative closest point, IPC, algorithm. The IPC algorithm may be a simulated annealing- (SA-) ICP algorithm or any other known ICP algorithm.

According to a second aspect, a computer program is provided. The computer program product comprises instructions which, when executed on at least one processor, cause the at least one processor to carry out the method described herein. The computer program may be stored on one or more computer readable recording media.

According to a third aspect, an apparatus for determining a surface registration between a first soft tissue surface defined based on mechanically acquired first surface data and a second soft tissue surface defined based on image data is provided. The apparatus is configured to obtain the first surface data, wherein the first surface data comprise a first set of points mechanically acquired by contacting the soft tissue with a pointing device, to apply a correction model on the first surface data to obtain corrected first surface data, wherein the correction model is configured to shift relative positions of two or more points in the first set, and to determine a first surface registration between the first and the second soft tissue surfaces based at least in part on the corrected first surface data.

The apparatus may be configured to carry out any of the method steps presented herein. The apparatus may comprise one or more processors and may be realized as a computing device.

According to a fourth aspect, a surgical system is provided. The surgical system comprises the apparatus according to the third aspect presented herein, and a pointing device configured to mechanically acquire the first set of points.

The surgical system may further comprise a tracker attached or attachable to the pointing device and a surgical tracking system configured to track the tracker.

The surgical system may be configured to be utilized for surgical navigation purposes. The surgical navigation may be based at least in part on the first surface registration or any subsequent surface registration.

### Brief Description of the Drawings

Further details, advantages and aspects of the present disclosure will become apparent from the following embodiments taken in conjunction with the drawings, wherein:
- Fig. 1: schematically illustrates two views of a mechanically acquired set of points registered to a facial skin surface that has been generated based on image data;
- Fig. 2A: schematically illustrates the mechanical acquisition of the points via a pointing device;
- Fig. 2B: schematically illustrates a registration error based on a pointing device-induced surface deformation;
- Fig. 2C: schematically illustrates an error for determining a location in a region of interest based on the registration comprising the registration error illustrated in Fig. 2B;
- Fig. 3: schematically illustrate a surgical system comprising a pointing device;
- Fig. 4: illustrates a flow diagram of a method for determining a surface registration that takes into account pointing device-induced surface deformations;
- Fig. 5: illustrates a correction vector that is to be applied to a mechanically acquired surface point;
- Fig. 6: illustrates an aspect of a linear soft tissue deformation model;
- Fig. 7: illustrates a block diagram of an exemplary iterative execution of the method of Fig. 4; and
- Fig. 8: schematically illustrates the first, uncorrected set of points of Fig. 1 and in a second set of corrected points each registered to the facial skin surface.

### Detailed Description

The following description is specifically related to surface registration for soft tissue in the facial region. It will be apparent that the present disclosure can also be implemented for other soft tissues and in other surgical contexts.

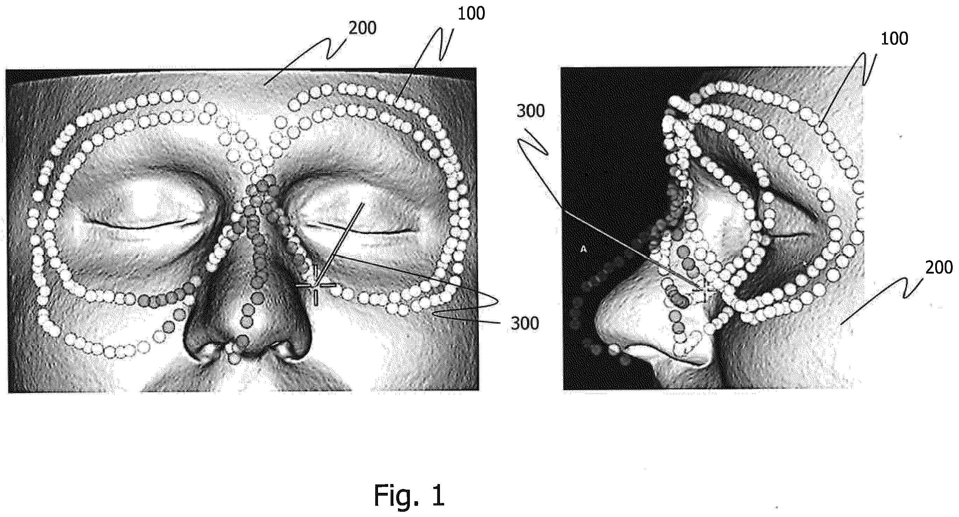

Fig. 1 illustrates mechanically acquired surface data, i.e., a set of points 100. The points 100 have been acquired by a pointing device and subsequently been registered to a soft tissue surface 200, i.e., a skin surface 200 of a face, defined based on image data (e.g., determined based on a CT scan). In Fig. 1, the skin surface 200 and the points 100 are shown once from the front of the face (on the left) and once from the side of the face (on the right).

The points 100 illustrated in Fig. 1 are depicted as circles. An accuracy of the match between the registered points 100 and the skin surface 200, i.e., a registration accuracy, is indicated by a brightness of the circles. The darker the circle, the farther away the corresponding registered point 100 is from the skin surface 200. In other words, the darker the circle, the lower the registration accuracy for the respective point 100.

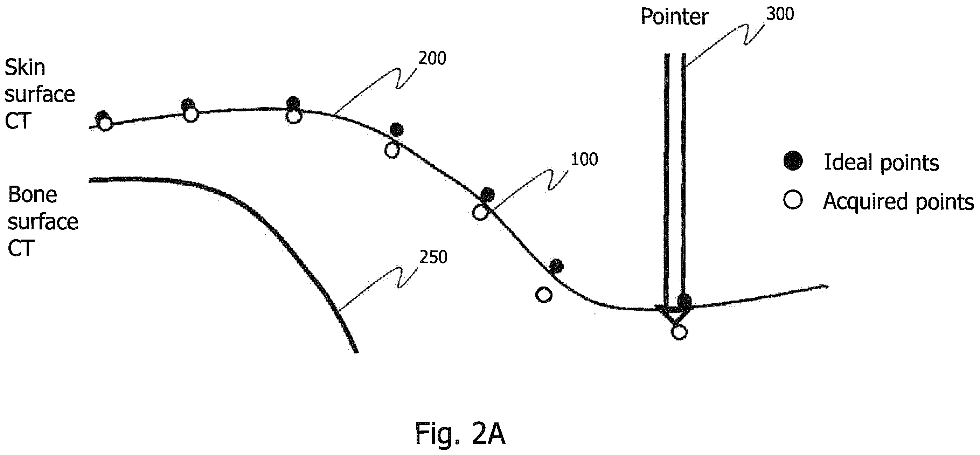

As can be gathered from Figs. 1 and 2A, most of the points 100 have been acquired at points 100 with an underlying bone tissue surface 250 located close to the skin surface 200, e.g., in the areas around the eyes, for example at the zygomatic bones. However, there are also points 100 acquired on soft tissue with no bone tissue surface 250 located close to the skin surface 200, e.g., like on the lower half of the nose. It is visible from Fig. 1 that the registration accuracy for the points 100 with no underlying bone tissue surface 250 located close to the skin surface 200 is low in comparison to the registration accuracy of the points 100 with an underlying bone surface 250 (not shown in Fig. 1) located close to the skin surface 200.

Further, a part of a pointing device 300, or simply pointer, utilized for mechanically acquiring the points 100 is schematically indicated in Figs. 1 and 2A. The pointer 300 may be a common pointer 300 known in the field of navigated surgery. A surgical tracker (not shown) may be attached to the pointer 300 for data acquisition purposes. The pointer 300 may comprise a button or other member for user input (i.e., for signalling to a data acquisition system when a data point is to be acquired).

Fig. 2A schematically illustrates the acquisition of the points 100 using the pointer 300. In Fig. 2A, a skin surface 200 and a bone surface 250 are schematically illustrated. The points 100 acquired via touching the skin surface 200 with the pointer 300 are illustrated as black circles with a white filling. For a comparison, points located on the skin surface 200 determined based on the CT scan are illustrated as black circles with a black filling and denoted as ideal points. As can be seen, the mechanically acquired points 100 are located in or below the skin surface 200, i.e., below the ideal points. The reason for the different locations of the mechanically acquired points 100 and the ideal points is due to a deformation of the skin surface 200 when being touched by the pointer 300, i.e., due to a pointing device-induced surface deformation resulting from applying a certain amount of force on the skin surface with each touching operation. As can be seen, a distance between the locations of the acquired points 100 and the ideal points generally increases with an increase of a distance between the skin surface 200 and the bone surface 250.

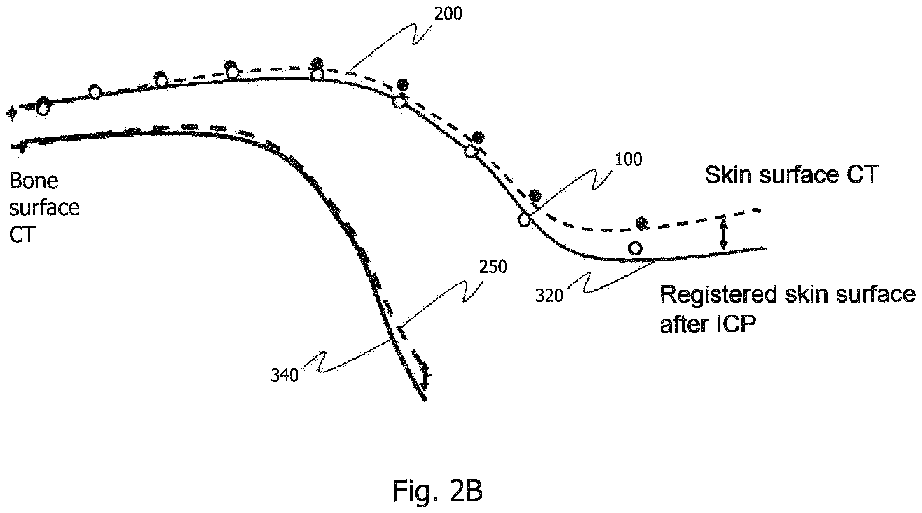

Due to the pointing device-induced surface deformation, a registration between the skin surface 200 as defined based on the acquired points 100 and a skin surface defined based on the CT scan comprises a registration error as schematically illustrated in Fig. 2B. In more detail, Fig. 2B illustrates the skin surface 200 and the bone surface 250 determined based on the CT scan as dotted lines. Further the mechanically determined points 100 and the ideal points are shown analogously to Fig. 2A. In addition, a skin surface 320 determined based on a registration between the mechanically determined points 100 and the skin surface 200 defined based on the CT scan as well as a bone surface 340 determined based on the registration are illustrated as black lines. The registration may be determined using a known registration technique, e.g., an ICP or SA-ICP algorithm. The resulting registration error is indicated as a distance between the surfaces 200, 250 determined based on the CT scan and the surfaces 320, 340 determined based on the registered mechanically acquired points 100, i.e., between the dotted lines and the black lines.

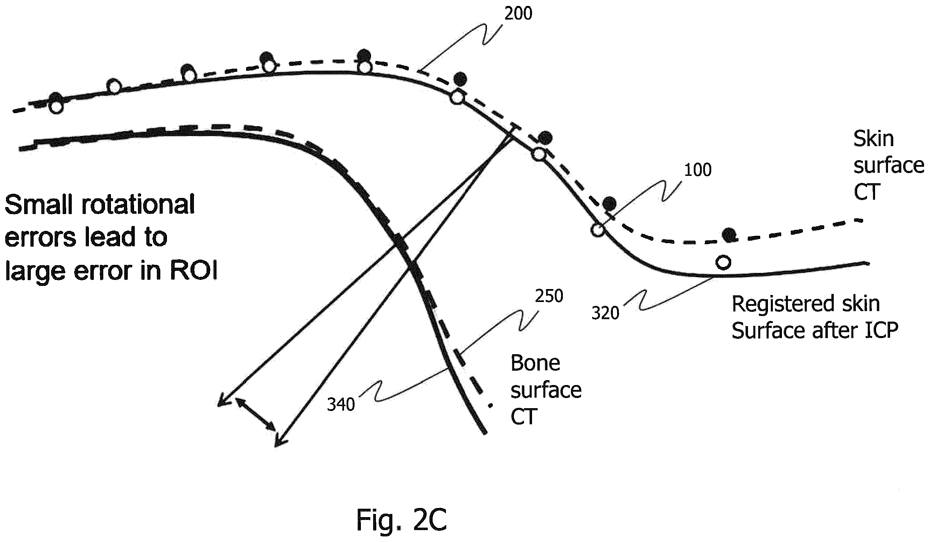

Fig. 2C schematically illustrates an error for determining a location in a region of interest, ROI, based on the registration comprising the registration error illustrated in Fig. 2B. In some surgical procedures, the surface registration of the mechanically acquired surface points 100 with the skin surface 200, as explained with reference to Figs. 1A to 2B, is used for determining the location of a brain tumour relative to the skin surface 200. The brain tumour may be located in the ROI located underneath the bone surface 250 as indicated in Fig. 2C. The location of the ROI is of interest for surgical navigation procedures, such as for guiding a surgical instrument to the ROI.

As can be seen in Fig. 2C, the position error in the ROI is multiple times larger than the registration error between the skin surface 200 and the surface 320 defined based on the registered points 100. A comparatively small registration error may thus lead to a relatively large error in determining the ROI (e.g., a tumour location), which poses a major health risk for a patient and may lead to a mistreatment.

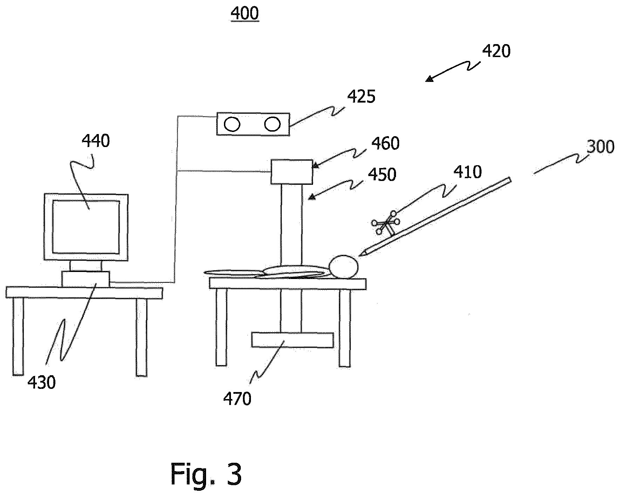

Fig. 3 schematically illustrates an exemplary surgical system 400 according to the present disclosure. The surgical system 400 is configured to determine an improved surface registration between a soft tissue surface defined based on mechanically acquired points 100 and the soft tissue surface 200, e.g., skin surface 200, defined based on image data (i.e., to eliminate or at least reduce a registration error, see Fig. 2C). The system 400 is specifically configured to enable acquiring the points 100 and the image data as well as process the acquired data. The system 400 may operate on the basis of patient image data for visualization, verification, navigation or other purposes.

The surgical system 400 illustrated in Fig. 3 comprises a pointing device 300 for mechanically acquiring the points 100. The pointing device 300 comprises an optical tracker 410 attached thereto. The tracker 410 is a commonly known optical tracker 410 and comprises, for example, four optically detectable fiducials. The tracker 410 may be an active or passive tracker (i.e., configured to actively emit or passively reflect light in the visible or infrared spectrum). The tracker 410 belongs to a surgical tracking system 420 that further comprise a mono or stereo camera 425, configured to detect the tracker 410 for tracking purposes. In other variants, the surgical system 400 may comprise an electromagnetic tracker and a field generator for tracking the position of the electromagnetic tracker. As explained above, the pointing device 300 may further comprise a user interface (e.g., a button) so signal to the surgical system (e.g., the tracking system 420 or the apparatus 430) when a data point is to be acquired.

The system 400 of Fig. 3 further comprises an apparatus 430 configured for receiving and processing the acquired data. In some variants, the apparatus 430 may be part of, or include, the surgical tracking system 420 and, optional, a surgical navigation system capable of outputting navigation instructions. In Fig. 3, the apparatus 430 is realized as a locally deployed computer system comprising one or more processors and memory storing program code for controlling operation of the one or more processors. Alternatively, the apparatus 430 may be realized in the form of a remote server or in the form of cloud computing resources. In other variants, the apparatus 430 may take the form of a tablet computer or other mobile device.

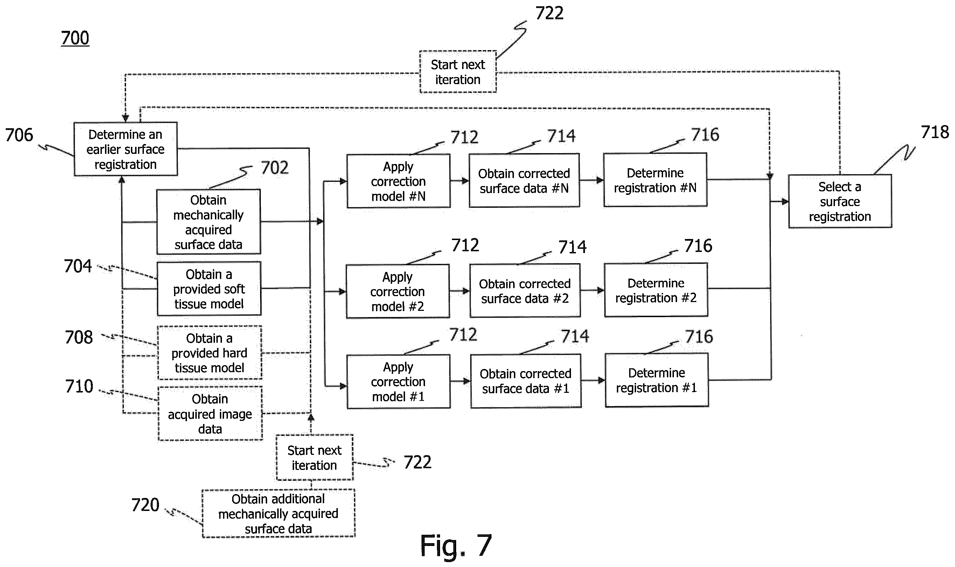

The system 400 of Fig. 3 further comprises an output device 440 configured to output information that has been derived by the apparatus 430 (e.g., for information, verification, modification or confirmation purposes, or for navigation purposes). In particular, the output device 440 is a display device configured to display the points 100 registered to the soft tissue surface 200 (e.g., as generally illustrated in Figs. 1 and 7). In such and other configurations, the output device 440 may further be configured to output navigation instructions for guiding a navigated surgical tool relative to an ROI of a patient to be treated. The output device 440 may be realized as, or comprise, a computer monitor, an augmented reality device (e.g., a head-mounted display), or a combination thereof.

Operation of the apparatus 430 is based on input data. The input data may comprise patient image data obtained on the basis of one or more surgical imaging techniques. For this reason, the surgical system 400 of Fig. 3 further comprises an imaging apparatus 450 configured to pre-operatively or intra-operatively acquire image data indicative of one or more ROIs of the patient. In the exemplary scenario illustrated in Fig. 3, the imaging apparatus 450 is a C-arm, for example with cone beam computer tomography (CBCT) imaging capabilities. In other scenarios, the imaging apparatus 450 is a conventional CT scanner that generates slice images, an EOS scanner or an X-ray apparatus. In still further scenarios, the imaging apparatus 450 utilizes a magneto resonance- (MR-) based imaging technique.

The exemplary CBCT-based imaging apparatus 450 of Fig. 3 comprises a radiation source 460 configured to generate a cone-shaped beam of radiation. Moreover, the imaging apparatus 450 has a flat-panel detector 470 configured to detect the radiation beam projected by the radiation source 460 through one or more of the ROIs of the patient. The detector 470 is configured to generate image data representative of two-dimensional projection images of the ROIs. As illustrated in Fig. 1, in some variants three-dimensional image data, e.g., data indicative of a facial skin surface 200, are derived from the resulting two-dimensional images using reconstruction (e.g., back-projection) techniques. The reconstruction may be performed either by the imaging apparatus 450 itself or by the apparatus 430. In such or other variants, the soft tissue surface 200 may be determined either by the imaging apparatus 450 or by the apparatus 430 based on predefined models, e.g., of a skin surface 200 and an underlying bone surface 250 or based on a combination of acquired image data and the models.

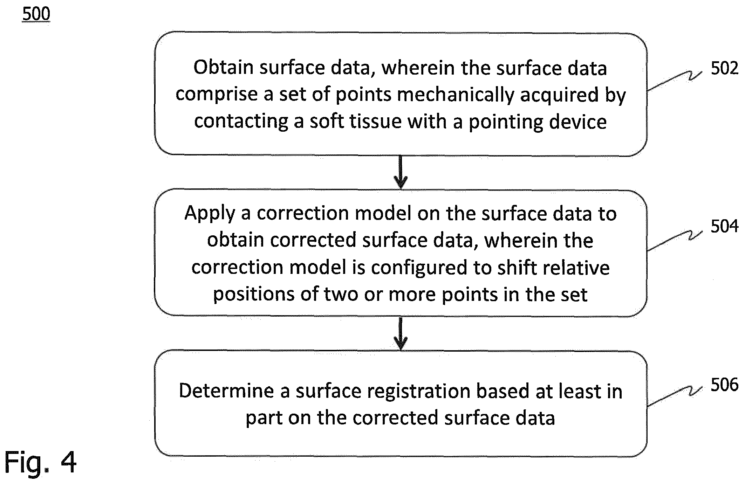

The imaging apparatus 450 or the apparatus 430 is further configured to process the image data acquired by the imaging apparatus 450 and the surface data, i.e., the set of points 100 mechanically acquired via use of the pointing device 300. The acquired data is processed for determining a surface registration between the soft tissue surface defined based on the mechanically acquired points 100 and the soft tissue surface 200 defined based on the acquired image data, as will now be explained in greater detail with reference to the flow diagram 500 of Fig. 4. The following explanations with reference to Fig. 4 are directed at an exemplary variant in which the determination of the surface registration is performed by the apparatus 430.

In an initial step 502, the acquired data, i.e., the acquired image data and the mechanically acquired surface data, are obtained by the apparatus 430. As an example, the apparatus 430 may obtain such data via a data interface.

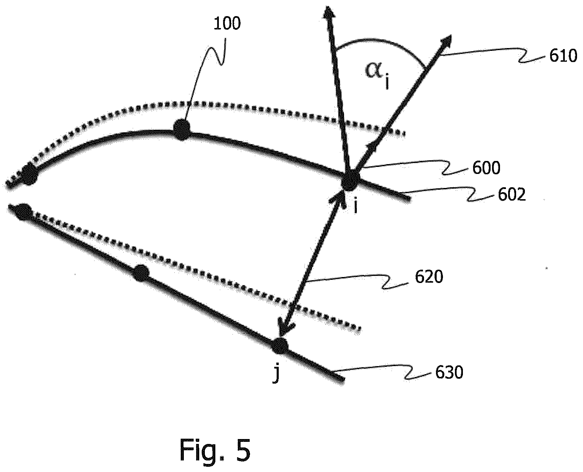

In step 504, a correction model is applied on the mechanically acquired surface data, i.e., the set of points 100. The correction model is configured to shift relative positions of two or more points 100 in the set. In some variants, the correction model is configured to determine a correction vector 600 for at least one point 100, as is shown in Fig. 5. The exemplary correction vector 600 shown in Fig. 5 is determined for point i out of the set of points 100 comprising a total of n points with i = 1, 2, ..., n, based on the following equation: ${\vec{correction}}_{i} = {w}_{force i} ∗ {w}_{distance i} ∗ {w}_{max - deformation} ∗ {\vec{dir}}_{surface i}$

W_{force i} defines a factor based on an angle αᵢ between a surface normal 610 of a soft tissue surface 602 at the respective point i and the pointing device 300, when the poiting device 300 touches the point i. One example for determining W_{force i} is as follows: ${w}_{force i} = cos \left({α}_{i}\right) with {α}_{i} = \frac{{\vec{dir}}_{surface i} ∗ {\vec{dir}}_{axis i}}{\left|{\vec{dir}}_{surface i}\right| \left|{\vec{dir}}_{axis i}\right|}$

In other variants, other equations may be used for determining W_{force i}.

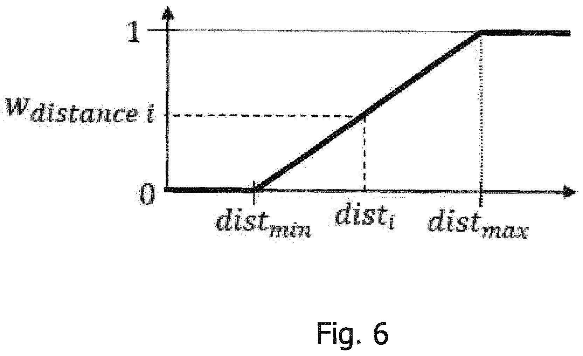

W_{distance i} defines a factor based on a distance 620 between point i and an associated point j on the underlying bone tissue surface 630. In the shown example, point j is defined as the point of the bone tissue surface 630 closest to point i. The distance 620 may be determined based on predetermined skin tissue and bone tissue models or based on medical image data or a combination thereof. W_{distance i} may further be defined based on a linear soft tissue deformation model as exemplarily shown in Fig. 6.

According to the linear soft tissue deformation model of Fig. 6, a minimum distance distₘᵢₙ between points i and j is required for any deformation to take place. Further, a maximum distance distₘₐₓ is defined, beyond which W_{distance i} does not increase any further. Between distₘᵢₙ and distₘₐₓ the factor defined by W_{distance i} increases linearly with the distance 620. In other variants other models than the shown example may be utilized for determining W_{distance i}, e.g., a non-linear model.

Returning to Fig. 5, W_{max-deformation} defines a factor based on a maximum allowed length of the correction vector 600. In some variants, the maximum length may be manually defined by a user. In other variants the maximum length may depend on a soft tissue model and/or a bone tissue model utilized for determining the distance 620 between points i and j.

The last factor in the exemplary equation, i.e., d̅i̅r̅_{surface i}, defines the extension direction of the correction vector 600. In the example of Fig. 5, the correction vector 600 is oriented parallel to a surface normal 610 of the soft tissue surface 602 at the respective point 100 and when touched by the pointing device 300.

In other variants, other equations may be used for determining the correction vector 600. Moreover, one or more factors in the above equation may be omitted, and one or more additional factors or linear terms may be added.

Applying the correction model thus generates corrected surface data in step 504. In step 506 a registration is determined based at least in part on the corrected surface data. The registration may be determined based on applying an ICP algorithm on the corrected surface data.

In some variants, additional surface data may be acquired by touching additional points on the soft tissue surface 200 with the pointing device 300. The additionally touched points may be corrected analogously to the points 100 obtained in step 502, as described with reference to step 504. The additionally touched points and the points 100 obtained in step 502 may thus form a base for determining a subsequent registration that is determined analogously to the registration determined in step 506. As such, the registration may continuously be updated as the user continues acquiring surface data.

In one variant, the correction model may be subsequently applied on the additionally touched points and a subsequent registration may be determined based on the corrected additionally acquired points and the corrected points 100 obtained in step 504 analogously to step 506. In another example, the additionally touched points and the points 100 obtained in step 502 may form a total of points on which the correction model is applied analogously to step 504, and a subsequent registration is determined on the corrected data analogously to step 506.

The steps of acquiring additional surface data and determining a subsequent surface registration may define an iterative process that may be continued until a predetermined registration accuracy is achieved or until a subsequent surface registration fails to provide a predefined improvement of the registration accuracy in comparison to a registration accuracy of a previous surface registration. The additional surface data may be acquired in parallel to the apparatus 430 performing the method 500.

A block diagram 700 directed at an exemplary variant of an iterative process is illustrated in Fig. 7.

Analogously to the first step 502 of the method 500 explained with reference to Fig. 4, mechanically acquired surface data is obtained in block 702. Further, at least one of a soft tissue model (e.g., a skin model) and image data, e.g., medical image data obtained as described herein with reference to Figs. 3 and 4, is obtained in block 704 and block 710, respectively. Further a hard tissue model (e.g., bone tissue model) may be obtained in block 708. Based on the obtained surface data and at least one of the soft tissue model and the medical image data, an earlier surface registration is determined in step 706 ("earlier" in comparison to a registration determined later, e.g., in block 718 and analogous to the first surface registration determined in step 506 of the method 500 explained with reference to Fig. 4). Additionally, the earlier surface registration may be determined based at least in part on the hard tissue model (e.g., bone tissue model) in step 706. In the exemplary variant illustrated in Fig. 7, the earlier surface registration is determined based on the obtained surface data and the soft tissue model. In some variants, the soft tissue model defines a soft tissue surface via a point cloud or meshes. The point cloud or meshes may be determined based on medical image data, e.g., CT-scans. In some variants, the accuracy of the soft tissue model may vary for different regions of the soft tissue surface. The soft tissue model accuracy may be optimized at predetermined points of the soft tissue surface or along a predetermined line on the soft tissue surface.

At least one correction model is applied 712 to the surface data under the use of the earlier surface registration, the surface data, and at least one of the soft tissue model, and the image data, and, optionally, the hard tissue model. The earlier surface registration and at least one of the soft tissue model and the image data, and, optionally, the hard tissue model are used for determining at least one input parameter for each of the correction models #1 to #N that are to be applied on the surface data obtained in block 712. In the variant shown in Fig. 7, the at least one input parameter is determined based on the earlier surface registration, the obtained surface data and the soft tissue model.

The different correction models #1 to #N are applied to obtain different versions of corrected surface data #1 to N#, as illustrated in blocks 712, and to determine different surface registrations #1 to #N based on the different corrected surface data #1 to #N, as illustrated in blocks 714. Based on, e.g., a comparison between the different surface registrations #1 to #N, and optionally the earlier surface registration, a surface registration may be selected as illustrated in block 718. For example, the surface registration selected according to block 718 may be the registration out of the registrations #1 to #N that has the highest registration accuracy. The surface registration determined in block 718 may then be used as the earlier surface registration for a next iteration 722 in case additional mechanically acquired surface data have been obtained 720 on which the correction models #1 to #N are to be applied in the next iteration 722.

Any of the registrations explained with reference to Fig. 7 may be determined using a known registration technique, e.g., an ICP or SA-ICP algorithm.

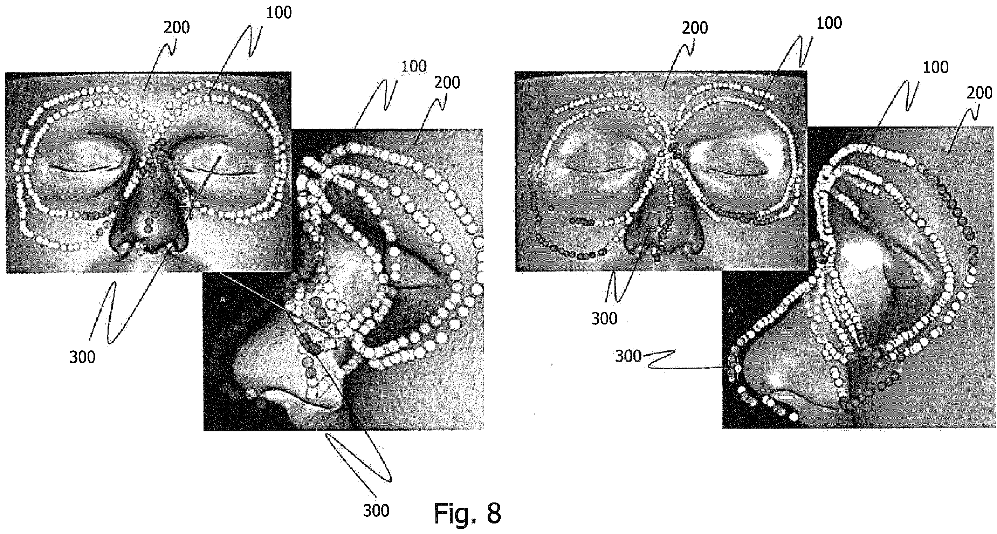

The registered corrected surface data may be displayed to a user on the output device 440 of Fig. 3, as is schematically illustrated in Fig. 8. In detail, Fig. 8 illustrates registered uncorrected surface data as shown in Fig. 1 on the left side and the registered corrected surface data on the right side. As can be seen, the registered corrected surface data provide a higher registration accuracy, in particular for facial areas with a comparatively larger distance 620 between the skin surface and an underlying bone tissue.

Since the technique described with reference to Figs. 4 to 8 significantly increase the registration accuracy for points located on soft tissue, a user is no longer required to touch skin surfaces with an underlying bone surface located close to the skin surface. Thus, the technique described herein facilitates data acquisition with a pointing device 300 and provides surface registrations with an improved registration accuracy, in particular for points located on soft tissue surface without an underlying hard tissue surface located close thereto. As a result, health risks for the patient are reduced, for example when the registration is used as a basis for generating navigation instructions for a surgeon or surgical robot.

## Claims

1. A computer-implemented method (500) for determining a surface registration between a first soft tissue surface (602) defined based on mechanically acquired first surface data and a second soft tissue surface (200) defined based on image data, the method comprising:
obtaining (502) the first surface data, wherein the first surface data comprise a first set of points (100) mechanically acquired by contacting the soft tissue with a pointing device (300);
applying (504) a correction model on the first surface data to obtain corrected first surface data, wherein the correction model is configured to shift relative positions of two or more points (100) in the first set; and
determining (506) a first surface registration between the first and the second soft tissue surfaces (602, 200) based at least in part on the corrected first surface data.

2. The method (500) of claim 1, wherein
the correction model is configured to determine a correction vector (600) for at least one of the points (100) in the first set, wherein the correction vector (600) defines a shift relative to one or more further points (100) in the first set.

3. The method (500) of claim 2, wherein
an earlier surface registration has been determined prior to the first surface registration and based at least in part on the first surface data, and
the correction vector (600) is determined based at least in part on the earlier surface registration.

4. The method (500) of claim 2 or 3, wherein
the correction vector (600) is oriented normal to the first soft tissue surface (602).

5. The method (500) of any of claims 2 to 4, wherein
obtaining (502) the first surface data comprises determining, for at least one of the points (100) in the first set, an angle between the pointing device (300) and a surface normal (610) of the first soft tissue surface (602) when the pointing device (300) contacts the soft tissue at the respective point (100); and wherein
the correction vector (600) is determined based at least in part on the angle determined for the point (100) associated with the respective correction vector (600).

6. The method (500) of any of claims 2 to 5, wherein
a maximum length of the correction vector (600) is predefined.

7. The method (500) of any of claims 2 to 6, wherein
at least one of the points (100) in the first set is associated with a respective point on a hard tissue surface (630) located beneath the soft tissue surface;
obtaining the first surface data comprises determining, for the at least one of the points (100) in the first set, a distance (620) between the respective point (100) in the first set and the associated point on the hard tissue surface (630); and wherein
the correction vector (600) is determined based at least in part on the distance (620) determined for the point (100) in the first set associated with the correction vector (600).

8. The method (500) of claim 7, wherein
the respective point on the hard tissue surface (630) is the point on the hard tissue surface (630) that is closest to the associated point (100) in the first set.

9. The method (500) of claim 7 or 8, wherein
determining the correction vector (600) is based at least in part on a linear soft tissue deformation model that receives the distance (620) as an input parameter.

10. The method (500) of claim 9, wherein
the linear soft tissue deformation model defines a minimum distance threshold below which no deformation takes place; and/or
the linear soft tissue deformation model defines a maximum distance threshold indicative of a maximum distance-based deformation.

11. The method (500) of any of claims 7 to 10, wherein
a soft tissue model and a hard tissue model are provided, and wherein the distance (620) is obtained based at least in part on the soft tissue model and the hard tissue model.

12. The method (500) of any preceding claim, wherein
multiple different correction models configured to shift relative positions of two or more points (100) in the first set are provided, and wherein the method (500) further comprises:
applying each of the different correction models to the first surface data to obtain different versions of the corrected first surface data; and wherein
the first surface registration is determined based at least in part on the different versions of the first surface data.

13. The method (500) of claim 12, wherein
a surface registration is determined for each of the different versions of the first surface data and the first surface registration is determined based on respective registration accuracies of the surface registrations determined for the different versions of the first surface data, wherein the respective registration accuracies are based on distances between each registered point and the second soft tissue surface for the respective registration.

14. A computer program comprising instructions which, when executed on at least one processor, cause the at least one processor to carry out the method (500) according to any one of claims 1 to 13, wherein the computer program is optionally stored on one or more computer readable media.

15. An apparatus (430) for determining a surface registration between a first soft tissue surface (602) defined based on mechanically acquired first surface data and a second soft tissue surface (200) defined based on image data, the apparatus being configured to:
obtain (502) the first surface data, wherein the first surface data comprise a first set of points (100) mechanically acquired by contacting the soft tissue with a pointing device (300);
apply (504) a correction model on the first surface data to obtain corrected first surface data, wherein the correction model is configured to shift relative positions of two or more points (100) in the first set; and
determine (506) a first surface registration between the first and the second soft tissue surfaces (602, 200) based at least in part on the corrected first surface data.

16. The apparatus (430) of claim 15, configured to carry out the method (500) of any of claims 2 to 13.

17. A surgical system (400) comprising
the apparatus (430) of claim 15 or 16; and
a pointing device (300) configured to mechanically acquire the first set of points (100).

18. The surgical system (400) of claim 17, further comprising:
a tracker (410) attached or attachable to the pointing device (300); and
a surgical tracking system (420) configured to track the tracker (300).

## Patentansprüche

1. Computerimplementiertes Verfahren (500) zum Bestimmen einer Oberflächenregistrierung zwischen einer ersten Weichteilgewebeoberfläche (602), die basierend auf mechanisch erfassten ersten Oberflächendaten definiert ist, und einer zweiten Weichteilgewebeoberfläche (200), die basierend auf Bilddaten definiert ist, wobei das Verfahren Folgendes umfasst:
Erhalten (502) der ersten Oberflächendaten, wobei die ersten Oberflächendaten einen ersten Satz von Punkten (100) umfassen, die mechanisch durch Kontaktieren des Weichteilgewebes mit einer Zeigevorrichtung (300) erfasst werden;
Anwenden (504) eines Korrekturmodells auf die ersten Oberflächendaten, um korrigierte erste Oberflächendaten zu erhalten, wobei das Korrekturmodell dazu eingerichtet ist, relative Positionen von zwei oder mehr Punkten (100) in dem ersten Satz zu verschieben; und
Bestimmen (506) einer ersten Oberflächenregistrierung zwischen der ersten und der zweiten Weichteilgewebeoberfläche (602, 200) mindestens teilweise basierend auf den korrigierten ersten Oberflächendaten.

2. Verfahren (500) nach Anspruch 1, wobei
das Korrekturmodell dazu eingerichtet ist, einen Korrekturvektor (600) für mindestens einen der Punkte (100) in dem ersten Satz zu bestimmen, wobei der Korrekturvektor (600) eine Verschiebung relativ zu einem oder mehreren weiteren Punkten (100) in dem ersten Satz definiert.

3. Verfahren (500) nach Anspruch 2, wobei
vor der ersten Oberflächenregistrierung und mindestens teilweise basierend auf den ersten Oberflächendaten eine frühere Oberflächenregistrierung bestimmt wurde, und
der Korrekturvektor (600) mindestens teilweise basierend auf der früheren Oberflächenregistrierung bestimmt wird.

4. Verfahren (500) nach Anspruch 2 oder 3, wobei
der Korrekturvektor (600) normal zu der ersten Weichteilgewebeoberfläche (602) ausgerichtet ist.

5. Verfahren (500) nach einem der Ansprüche 2 bis 4, wobei
das Erhalten (502) der ersten Oberflächendaten ein Bestimmen eines Winkels zwischen der Zeigevorrichtung (300) und einer Oberflächennormalen (610) der ersten Weichteilgewebeoberfläche (602) für mindestens einen der Punkte (100) in dem ersten Satz umfasst, wenn die Zeigevorrichtung (300) das Weichteilgewebe an dem jeweiligen Punkt (100) kontaktiert; und wobei
der Korrekturvektor (600) mindestens teilweise basierend auf dem Winkel bestimmt wird, der für den Punkt (100) bestimmt wurde, der dem jeweiligen Korrekturvektor (600) zugeordnet ist.

6. Verfahren (500) nach einem der Ansprüche 2 bis 5, wobei eine maximale Länge des Korrekturvektors (600) vordefiniert ist.

7. Verfahren (500) nach einem der Ansprüche 2 bis 6, wobei
mindestens einer der Punkte (100) in dem ersten Satz einem jeweiligen Punkt auf einer Hartgewebeoberfläche (630) zugeordnet ist, die sich unter der Weichteilgewebeoberfläche befindet;
das Erhalten der ersten Oberflächendaten für den mindestens einen der Punkte (100) in dem ersten Satz ein Bestimmen eines Abstands (620) zwischen dem jeweiligen Punkt (100) in dem ersten Satz und dem zugeordneten Punkt auf der Hartgewebeoberfläche (630) umfasst; und
wobei der Korrekturvektor (600) mindestens teilweise basierend auf dem Abstand (620) bestimmt wird, der für den dem Korrekturvektor (600) zugeordneten Punkt (100) in dem ersten Satz bestimmt wurde.

8. Verfahren (500) nach Anspruch 7, wobei
der jeweilige Punkt auf der Hartgewebeoberfläche (630) der Punkt auf der Hartgewebeoberfläche (630) ist, der dem zugeordneten Punkt (100) in dem ersten Satz am nächsten ist.

9. Verfahren (500) nach Anspruch 7 oder 8, wobei
das Bestimmen des Korrekturvektors (600) mindestens teilweise auf einem linearen Weichteilgewebedeformationsmodell basiert, das den Abstand (620) als einen Eingabeparameter empfängt.

10. Verfahren (500) nach Anspruch 9, wobei
das lineare Weichteilgewebedeformationsmodell einen minimalen Abstandsschwellenwert definiert, unterhalb dessen keine Deformation stattfindet; und/oder
das lineare Weichteilgewebedeformationsmodell einen maximalen Abstandsschwellenwert definiert, der eine maximale abstandsbasierte Deformation angibt.

11. Verfahren (500) nach einem der Ansprüche 7 bis 10, wobei
ein Weichteilgewebemodell und ein Hartgewebemodell bereitgestellt werden, und wobei der Abstand (620) mindestens teilweise basierend auf dem Weichteilgewebemodell und dem Hartgewebemodell erhalten wird.

12. Verfahren (500) nach einem der vorhergehenden Ansprüche, wobei
mehrere verschiedene Korrekturmodelle, die dazu eingerichtet sind, relative Positionen von zwei oder mehr Punkten (100) in dem ersten Satz zu verschieben, bereitgestellt werden, und wobei das Verfahren (500) ferner Folgendes umfasst:
Anwenden jedes der verschiedenen Korrekturmodelle auf die ersten Oberflächendaten, um verschiedene Versionen der korrigierten ersten Oberflächendaten zu erhalten; und wobei
die erste Oberflächenregistrierung mindestens teilweise basierend auf den verschiedenen Versionen der ersten Oberflächendaten bestimmt wird.

13. Verfahren (500) nach Anspruch 12, wobei
für jede der verschiedenen Versionen der ersten Oberflächendaten eine Oberflächenregistrierung bestimmt wird und die erste Oberflächenregistrierung basierend auf jeweiligen Registrierungsgenauigkeiten der für die verschiedenen Versionen der ersten Oberflächendaten bestimmten Oberflächenregistrierungen bestimmt wird, wobei die jeweiligen Registrierungsgenauigkeiten auf Abständen zwischen jedem registrierten Punkt und der zweiten Weichteilgewebeoberfläche für die jeweilige Registrierung basieren.

14. Computerprogramm, umfassend Anweisungen, die, wenn sie auf mindestens einem Prozessor ausgeführt werden, den mindestens einen Prozessor dazu veranlassen, das Verfahren (500) nach einem der Ansprüche 1 bis 13 durchzuführen, wobei das Computerprogramm optional auf einem oder mehreren computerlesbaren Medien gespeichert ist.

15. Vorrichtung (430) zum Bestimmen einer Oberflächenregistrierung zwischen einer ersten Weichteilgewebeoberfläche (602), die basierend auf mechanisch erfassten ersten Oberflächendaten definiert ist, und einer zweiten Weichteilgewebeoberfläche (200), die basierend auf Bilddaten definiert ist, wobei die Vorrichtung eingerichtet ist zum:
Erhalten (502) der ersten Oberflächendaten, wobei die ersten Oberflächendaten einen ersten Satz von Punkten (100) umfassen, die mechanisch durch Kontaktieren des Weichteilgewebes mit einer Zeigevorrichtung (300) erfasst werden;
Anwenden (504) eines Korrekturmodells auf die ersten Oberflächendaten, um korrigierte erste Oberflächendaten zu erhalten, wobei das Korrekturmodell dazu eingerichtet ist, relative Positionen von zwei oder mehr Punkten (100) in dem ersten Satz zu verschieben; und
Bestimmen (506) einer ersten Oberflächenregistrierung zwischen der ersten und der zweiten Weichteilgewebeoberfläche (602, 200) mindestens teilweise basierend auf den korrigierten ersten Oberflächendaten.

16. Vorrichtung (430) nach Anspruch 15, die dazu eingerichtet ist, das Verfahren (500) nach einem der Ansprüche 2 bis 13 durchzuführen.

17. Chirurgisches System (400), umfassend:
die Vorrichtung (430) nach Anspruch 15 oder 16; und
eine Zeigevorrichtung (300), die dazu eingerichtet ist, den ersten Satz von Punkten (100) mechanisch zu erfassen.

18. Chirurgisches System (400) nach Anspruch 17, ferner umfassend:
einen Tracker (410), der an der Zeigevorrichtung (300) befestigt oder befestigbar ist; und
ein chirurgisches Trackingsystem (420), das dazu eingerichtet ist, den Tracker (410) zu verfolgen.

## Revendications

1. Procédé mis en œuvre par ordinateur (500) pour déterminer un enregistrement de surface entre une première surface de tissu mou (602), définie à partir de premières données de surface acquises mécaniquement, et une deuxième surface de tissu mou (200), définie à partir de données d'image, le procédé comprenant:
l'acquisition (502) des premières données de surface, les premières données de surface comprenant un premier ensemble de points (100) acquis mécaniquement par contact entre le tissu mou et un dispositif de pointage (300);
l'application (504) d'un modèle de correction aux premières données de surface afin d'obtenir des premières données de surface corrigées, le modèle de correction étant configuré pour décaler les positions relatives d'au moins deux points (100) dans le premier ensemble; et
la détermination (506) d'un premier enregistrement de surface entre les première et deuxième surfaces de tissu mou (602, 200) sur la base d'au moins en partie des premières données de surface corrigées.

2. Procédé (500) selon la revendication 1, dans lequel
le modèle de correction est configuré pour déterminer un vecteur de correction (600) pour au moins l'un des points (100) dans le premier ensemble, le vecteur de correction (600) définissant un décalage par rapport à un ou plusieurs autres points (100) du premier ensemble.

3. Procédé (500) selon la revendication 2, dans lequel
un enregistrement de surface antérieur a été déterminé avant le premier enregistrement de surface et repose, au moins en partie, sur les premières données de surface, et
le vecteur de correction (600) est déterminé sur la base, au moins en partie, sur l'enregistrement de surface antérieur.

4. Procédé (500) selon la revendication 2 ou 3, dans lequel le vecteur de correction (600) est orienté perpendiculairement à la première surface de tissu mou (602).

5. Procédé (500) selon l'une quelconque des revendications 2 à 4, dans lequel
l'obtention (502) des premières données de surface comprend la détermination, pour au moins l'un des points (100) dans le premier ensemble, d'un angle entre le dispositif de pointage (300) et une normale (610) à la surface du premier tissu mou (602) lorsque le dispositif de pointage (300) entre en contact avec le tissu mou au niveau du point respectif (100); et dans lequel
le vecteur de correction (600) est déterminé sur la base, au moins en partie, de l'angle déterminé pour le point (100) associé au vecteur de correction (600) correspondant.

6. Procédé (500) selon l'une quelconque des revendications 2 à 5, dans lequel une longueur maximum du vecteur de correction (600) est prédéfini.

7. Procédé (500) selon l'une quelconque des revendications 2 à 6, dans lequel
au moins l'un des points (100) dans le premier ensemble est associé à un point correspondant situé sur une surface de tissu dur (630) située sous la surface de tissu mou;
l'obtention des premières données de surface comprend la détermination, pour ledit au moins un des points (100) de l'ensemble premier, d'une distance (620) entre le point respectif (100) de l'ensemble premier et le point associé sur la surface de tissu dur (630) ; et dans lequel
le vecteur de correction (600) est déterminé sur la base, au moins en partie, de la distance (620) déterminée pour le point (100) de l'ensemble 1 associé au vecteur de correction (600).

8. Procédé (500) selon la revendication 7, dans lequel
le point correspondant sur la surface du tissu dur (630) est le point de la surface de tissu dur (630) qui est le plus proche du point associé (100) dans le premier ensemble.

9. Procédé (500) selon la revendication 7 ou 8, dans lequel
la détermination du vecteur de correction (600) repose, au moins en partie, sur un modèle linéaire de déformation de tissu mou qui reçoit la distance (620) comme paramètre d'entrée.

10. Procédé (500) selon la revendication 9, dans lequel
le modèle de déformation linéaire de tissu mou définit un seuil de distance minimale en dessous duquel aucune déformation ne se produit; et/ou
le modèle de déformation linéaire de tissu mou définit un seuil de distance maximale indiquant une déformation en fonction de la distance maximale.

11. Procédé (500) selon l'une quelconque des revendications 7 à 10, dans lequel
un modèle de tissu mou et un modèle de tissu dur sont fournis, et dans lequel la distance (620) est obtenue en se basant, au moins en partie, sur le modèle de tissu mou et le modèle de tissu dur.

12. Procédé selon l'une quelconque revendication précédente, dans lequel
plusieurs modèles de correction différents, configurés pour décaler les positions relatives d'au moins deux points (100) dans le premier ensemble, sont fournis, et le procédé (500) comprenant en outre:
l'application de chacun des différents modèles de correction aux premières données de surface afin d'obtenir différentes versions des premières données corrigées de surface; et dans lequel
le premier enregistrement de surface est déterminé en fonction, au moins en partie, des différentes versions des premières données de surface.

13. Procédé (500) selon la revendication 12, dans lequel
un enregistrement de surface est déterminé pour chacune des différentes versions des premières données de surface, et le premier enregistrement de surface est déterminé sur la base des précisions d'enregistrement respectives des enregistrements de surface déterminées pour les différentes versions des premières données de surface, les précisions d'enregistrement respectives étant fondées sur les distances entre chaque point enregistré et la deuxième surface de tissu mou pour l'enregistrement correspondant.

14. Programme informatique comprenant des instructions qui, lorsqu'elles sont exécutées sur au moins un processeur, amènent l'au moins un processeur à exécuter le procédé (500) selon l'une quelconque des revendications 1 à 13, le programme informatique étant éventuellement stocké sur un ou plusieurs supports lisibles par ordinateur.

15. Appareil (430) pour déterminer un enregistrement de surface entre une première surface de tissu mou (602), définie à partir de premières données de surface acquises mécaniquement, et une deuxième surface de tissu mou (200), définie à partir de données d'image, l'appareil étant configuré pour:
obtenir (502) les premières données de surface, les premières données de surface comprenant un premier ensemble de points (100) acquis mécaniquement par contact entre le tissu mou et un dispositif de pointage (300);
appliquer (504) un modèle de correction aux premières données de surface afin d'obtenir des premières données de surface corrigées, le modèle de correction étant configuré pour décaler les positions relatives d'au moins deux points (100) dans le premier ensemble; et
déterminer (506) un premier enregistrement de surface entre les première et deuxième surfaces de tissu mou (602, 200) sur la base d'au moins en partie des premières données de surface corrigées.

16. Appareil (430) selon la revendication 15, configuré en outre pour exécuter le procédé (500) selon l'une quelconque des revendications 2 à 13.

17. Système chirurgical (400) comprenant
l'appareil (430) selon la revendication 15 ou 16; et
un dispositif de pointage (300) configuré pour acquérir mécaniquement le premier ensemble de points (100).

18. Système chirurgical (400) selon la revendication 17, comprenant en outre:
un dispositif de suivi (410) fixé ou pouvant se fixer au dispositif de pointage (300); et
un système de suivi chirurgical (420) configuré pour suivre le dispositif de suivi (410).
